# EUROPEAN PATENT APPLICATION

(11) **EP 2 977 464 A1**
(43) Date of publication of application: **27.01.2016**
(21) Application number: 14767593.8
(22) Date of filing: 19.03.2014
(51) Int. Cl.: C12Q 1/68, C12N 15/09, G01N 33/574

(54) **METHOD FOR PREDICTING SENSITIVITY TO EGFR INHIBITOR**

(30) Priority: 19.03.2013 JP 2013057033
(71) Applicant: Toppan Printing Co., Ltd., Tokyo 110-0016 (JP)
(72) Inventor: KITANO Shiro, Tokyo 110-0016 (JP); YAMADA Takeshi, Tokyo 113-8603 (JP)
(74) Representative: TBK
(86) International application number: PCT/JP2014/057556
(87) International publication number: WO 2014/148557

(57) **Abstract**

A method for predicting sensitivity to an EGFR inhibitor includes: (a) determining whether there is a KRAS gene-derived nucleic acid or a protein thereof in a blood sample which has been collected from a subject, and whether the KRAS gene-derived nucleic acid or the protein thereof in the blood sample is wild type or mutant; and (b) determining that there is a high possibility that a tumor of the subject is sensitive to an EGFR inhibitor when a wild type KRAS gene-derived nucleic acid or a protein thereof is detected and no mutant KRAS gene-derived nucleic acid or a protein thereof is detected in the blood sample in the process (a), and determining that there is a high possibility that the tumor of the subject is not sensitive to the EGFR inhibitor in a case where the mutant KRAS gene-derived nucleic acid or the protein thereof is detected in the blood sample.

SEQUENCE LISTING
<110>  TOPPAN Printing Co., Ltd.
<120>  Method for prediction of sensitivity to EGFR inhibitor drug
<130>  P20130167
<160>  6
<170>  PatentIn version 3.1

<210>  1
<211>  188
<212>  PRT
<213>  Homo sapiens
<220>
<223>  KRAS
<400>  1


<210>  2
<211>  3360
<212>  DNA
<213>  Homo sapiens
<400>  2
tcgcttgagg ccaggagttg gagaccagcg tactcaacat agtgagacct tgttataaag      60
aaaaaaaaaa tccaggatta aaaaaaatct ttgatttgtt tgggatttat taatatttac     120
cgtattggaa attaaaacaa ttttttaaaa tgtattcatt taaaaataat aagcccatta     180
cttggtaaca tgaataaaat attttatgaa aaataactat tttccaaaac aaaaccaaaa     240
cttagaaaag tggtattgtt tcacacttca gtaaatctct ttaatgatgt ggcttaatag     300
aagatatgga ttcttatatc tgcatctgca ttcaatctat tatgatcaca catctggaaa     360
acttgtgaaa gaatgggagt taaaagggta aaggacatct taatgttatt atgaaaacag     420
ttttgacctc ttgcacacca gaaaagtctt agtaacctga ggggttccta gaccacattt     480
tgagaactgt tttaggctat gcaaactggt tggggggagg ttggggtagg cagagagcta     540
gaagatacat tttagtgtaa ttctcctcat ctattcctaa ttgctttggc ctacatttga     600
aataaagcgt ggaggcaaac gggataagat acatgtttgt agtggttgtt aacttcaccc     660
tagacaagca gccaataagt ctaggtagag cagagtaagg cggggaacta tgccgtgacc     720
gtgtgtgata caatttttct agcctgtggt gctttttgcg gcagggctta ggagtaaggt     780
tagtatgtta tcatttggga aaccaaatta ttattttggg tcttcagtca attatgatgc     840
tgtgtatatt tagtgtttat ctacaatata tgcacattca ttaatttgga gctactcatc     900
ctataataaa tagttgtgca tttactccca tttttttctg catttctctc cttatttata     960
attatgtgtt acatgaggga aaggaggtga aattaaacat tcatattatt tcaaaaaatt    1020

tgaaacaact aactaaaaaa tatgttttat tttctgtatg gtgtttgtta tacaatctgt 1080
caatattcat gcacctcttg ggagacagtg tatgaaaagc aaagagtaac agtcacatgg 1140
attactgatt actgagatat attcacttgc atcttttttt ttttttgaga cggagtggct 1200
ctgtcgccca ggctggagtg cagtggcgtg atctcggctc actgcaagct ccgcctcctg 1260
ggttcacgcc attcttctgc ctcagcctcc caagtagctg ggactacagg cgcccgccac 1320
cacgcccggc taattttttt atatttttag tagagacggg gtttcaccgg gttagccagg 1380
atggtcttga tctcctgacc tcgtgatcca ccctcctcgg cctcccaaag tgctaggatt 1440
ataggcgtga gccaccgtgc ccggctcact tgcatctctt aacagctgtt ttcttactaa 1500
aaacagtgtt tatctctaat ctttttgttt gtttgtttgt tttgagatgg agtcttactc 1560
cgtcacccaa tctggagtgc agtggcgtga tctgggctca ctgcaacctc tgcctcccgg 1620
gttcaagtga ttctccttcc tcagcctccc cagtagctag gactacagga gagcgccacc 1680
acgcctgatt aatttttgta tttttagtag agagagggtt tcaccatatt ggccaggctg 1740
gtcttgaact cctggcctca ggtgatccac ccgccttggc ctctgaaagt gctgggatta 1800
caggcatgag ccgccgcacc cggctttcta atctttatct ttttttgtgc agcggtgata 1860
caggattatg tattgtactg aacagttaat tcggagttct cttggttttt agctttattt 1920
tccccagaga tttttttttt tttttttttt tttgagacgg agtcttgctc tatcgccagg 1980
ctggagtgca gtggcgccat ctcggctcat tgcaacctcg gactcctatt ttccccagag 2040
atatttcaca cattaaaatg tcgtcaaata ttgttcttct ttgcctcagt gtttaaattt 2100
ttatttcccc atgacacaat ccagctttat ttgacactca ttctctcaac tctcatctga 2160
ttcttactgt taatatttat ccaagagaac tactgccatg atgctttaaa agtttttctg 2220
tagctgttgc atattgactt ctaacactta gaggtggggg tccactagga aaactgtaac 2280
aataagagtg gagatagctg tcagcaactt ttgtgagggt gtgctacagg gtgtagagca 2340
ctgtgaagtc tctacatgag tgaagtcatg atatgatcct ttgagagcct ttagccgccg 2400
cagaacagca gtctggctat ttagatagaa caacttgatt ttaagataaa agaactgtct 2460
atgtagcatt tatgcatttt tcttaagcgt cgatggagga gtttgtaaat gaagtacagt 2520
tcattacgat acacgtctgc agtcaactgg aattttcatg attgaatttt gtaaggtatt 2580
ttgaaataat ttttcatata aaggtgagtt tgtattaaaa ggtactggtg gagtatttga 2640
tagtgtatta accttatgtg tgacatgttc taatatagtc acattttcat tatttttatt 2700
ataaggcctg ctgaaaatga ctgaatataa acttgtggta gttggagctg gtggcgtagg 2760
caagagtgcc ttgacgatac agctaattca gaatcatttt gtggacgaat atgatccaac 2820
aatagaggta aatcttgttt taatatgcat attactggtg caggaccatt ctttgataca 2880
gataaaggtt tctctgacca ttttcatgag tacttattac aagataatta tgctgaaagt 2940
taagttatct gaaatgtacc ttgggtttca agttatatgt aaccattaat atgggaactt 3000
tactttcctt gggagtatgt cagggtccat gatgttcact ctctgtgcat tttgattgga 3060
agtgtatttc agagtttcgt gagagggtag aaatttgtat cctatctgga cctaaaagac 3120
aatcttttta ttgtaacttt tatttttatg ggtttcttgg tattgtgaca tcatatgtaa 3180
aggttagatt taattgtact agtgaaatat aattgtttga tggttgattt ttttaaactt 3240
catcagcagt attttcctat cttcttctca acattagaga acctacaact accggataaa 3300
ttttacaaaa tgaattattt gcctaaggtg tggtttatat aaaggtacta ttaccaactt 3360

<210> 3
<211> 3480
<212> DNA
<213> Homo sapiens
<400> 3
   ttttaataga gatggggttt caccatgttg gccaggatgt tcttgatctc ctgacctcat 60
   gatccgccca cctcggcctc ccaaagtgtt gggattgcaa gtgtgagcca ccgcgcctag 120
   accatggtag ttaattttaa gtgttcaatt cagtgacctt aagtgtgttc ataatgttgt 180
   gcaaccatca ccatgttgtc taaccattag cactatctgt tttgagaact tttttttatc 240
   atcccaaatt agaattctgt acctgtcaaa tagtccccag taatcctccc tcccccagcc 300
   cctggtaatc tgtagtctac ttttcgtctt tttgaatttg cctattttag gttcctcata 360
   taagtggaat tatgtggtat ttgtcctttt gtgttggctt acttcattta gcataatgtt 420
   ttcaaggttc atctgtgttg tagcatgtat atacaggttg aagcatccgt tatccaaaat 480
   ggttgtgacc agaagtggtt tggatttcag attttttttt tggattttgg aatattcata 540
   gatacttaac tggttcagca tccctcgtcc aaaaatccaa aatcagatgg agctcagtgg 600
   ctcatgcttg taatcccaac acgttgggtg gccaaggcag gaggatcgct tgagcccagg 660
   agttcaacca gcctgagcaa cacaagaccc tatctctcca aaaaaaaaaa aaaaaaaaaa 720
   aagatgaaag aaaaaaaaat ccaaaatcaa atgctccagt gagcatttcc ttttagcatc 780
   atgtcaggct ctaaaagtta caggttttgg agcattttgg atttcagatt tttggattaa 840
   cctgcattaa tgctcaacct atatgaaatt ttattccttt ttatggctga ataatgttcc 900
   actgtatgta tatactacat tttgtttatc cattcatctg ttaacagaca cttaagttat 960
   ttccacattt tgggtattat aaatagtgct gctgcgaaca ttggtgtaca tgtatctgtt 1020
   tgagtccctg tttttagtta ttttggttat atacctagga atggaattgc tgatcatatg 1080
   gtaattctgt gtttaacttt ttgaggaact accactgttt tccacaatgg catcaccatt 1140
   ttacattccc accagcaatg cacaaagatt tcagtgtctg tatccttgct aacacttatt 1200
   ttccattttt tgagtttttt tgttttgttt ttttaataat agccaatcct aatgggtatg 1260
   tggtagcatc tcatggtttt gattttattt tcctgactat tgatgatgtt gagcatcttt 1320
   tcaggtgctt agtggccatt tgtccgtcat ctttggagca ggaacaatgt cttttcaagt 1380
   cctttgccca tttttaaatt gaattttttg ttgttgagtt gtatataaca ccttttttga 1440
   agtaaaaggt gcactgtaat aatccagact gtgtttctcc cttctcagga ttcctacagg 1500
   aagcaagtag taattgatgg agaaacctgt ctcttggata ttctcgacac agcaggtcaa 1560
   gaggagtaca gtgcaatgag ggaccagtac atgaggactg ggagggcttt tctttgtgta 1620
   tttgccataa ataatactaa atcatttgaa gatattcacc attataggtg ggtttaaatt 1680
   gaatataata agctgacatt aaggagtaat tatagttttt attttttgag tctttgctaa 1740
   tgccatgcat ataatattta ataaaaattt ttaaataatg tttatgaggt aggtaatatc 1800
   cctgttttat aaatgaagtt cttgggggat tagagcagtg gagtaacttg ctccagactg 1860
   catcggtagt ggtggtgctg ggattgaaac ctaggcctgt ttgactccac agccttctgt 1920
   actcttgact attctacaaa agcaagactt taaacttttt agatacatca ttaaaaaaga 1980
   aaaccataaa aaagaatatg aaaagatgat ttgagatggt gtcactttaa cagtcttaaa 2040
   agcaatcgtg tgtatagcat agaattgctt ggattggata aacagtggca ttatatattt 2100
   taaaaaataa aagttttgaa agattgaaga atttgggcat tacagttctc ttaaatctga 2160
   caaagctgca taaaactatt aaaataatca ttattatact attttatatt ctatttcttt 2220
   gagggtttag ttttccaaaa actacatatt aagcaaatga atcactcagt ggctatgtca 2280
   tataataacg agttagccta gttataagaa gtttaacatt ttatttaaga acattgttac 2340
   agcatgttta ctgtatagtc tagtaataga ggaaaagaca tttgggtggg tggtagtggt 2400
   agtattttta tagaggagtt accaaatttc agctctatta tccaagttta cccagctaat 2460
   ggtgttcgga accgggaatt tgagccaatt ctgactctgt tgtctgctct gctccttctt 2520
   ttgtgctgtg tctttgaaag tcacctaaaa ttgtgaggga atgtaatttc accccaaatt 2580
   tagagtttat gcacttgtta tattgaaaat gattaacatg tagaagggct tttaatggaa 2640
   taagtggtgt agtaacttca gtgttgccta cctagaaatc aaaatctttc tagttgtcca 2700
   ctttgttttt tgaaaaagta atatgaaaat tatgttaatg ctttaattca ggtttttgta 2760
   aaatattttt tatctttaca catttaacat acgtttctaa aattatagtc tgttatatag 2820
   cactttgggt ctagaatttt tcagtagttt ctgttttact attatgatct acctgcatat 2880
   taacctatta ggttatagtt ttactatact tctaggtatt tgatcttttg agagagatac 2940
   aaggtttctg tttaaaaagg taaagaaaca aaataactag tagaagaagg aaggaaaatt 3000
   tggtgtagtg gaaactagga attacattgt tttctttcag ccaaatttta tgacaaaagt 3060
   tgtggacagg ttttgaaaga tatttgtgtt actaatgact gtgctataac ttttttttct 3120
   ttcccagaga acaaattaaa agagttaagg actctgaaga tgtacctatg gtcctagtag 3180
   gaaataaatg tgatttgcct tctagaacag tagacacaaa acaggctcag gacttagcaa 3240
   gaagttatgg aattcctttt attgaaacat cagcaaagac aagacaggta agtaacactg 3300
   aaataaatac agatctgttt tctgcaaaat cataactgtt atgtcattta atatatcagt 3360
   ttttctctca attatgctat actaggaaat aaaacaatat ttagtaaatg tttttgtctc 3420
   ttgagagggc attgcttctt aatccagtgt ccatggtact gcttttggct ttggtttctt 3480

<210> 4
<211> 3540
<212> DNA
<213> Homo sapiens
<400> 4
tctacattga aaatttctct tcaattctga gcacatgtta acatttagaa ttcaagaggt 60
ggggattttt ttttcccatg gttacatata tatatatata tatatatata tatatatata 120
tatatatata tataaagaac agggcaacaa atttttgcgt tttctatttc ggtagtactt 180
ttaaaccatt atgtcatgtt tctaggttaa acgttgttgt atttgaagaa ttttactttg 240
gcagaatttt tttgaggatg tgtttatttc tggagaaagg tctcattaaa gaaagacaat 300
acccagaaag ccaacagaaa ttctgttact catttaatgc atttttctga caaaaattat 360
tgccagagag aacctgaatt ttgtttcaaa aatcatcttt gttttaaaaa tgactttttc 420
ttcaggtaaa ataaaataat ttcagttgct attatttaac ctgtttgtat gaagagttta 480
acatatagga aatgaataca taaagatagg aaggaattaa ttgttatatg tagtcatatg 540
tctcttaatg acagggatac tttctaagaa atacattgtt aggtgatttt gtcattgtgc 600
aaacatcata gaatatactt acacaaacct tggtagtata acctactata cacctgggat 660
atgtagtata gtctcttgcc ccagggatac aaacctgtac agtatgtaac tgtactaatg 720
actataaggc aattgttaac acaatggtaa gttttgtgtg tctaaaccta cacttgggct 780
accctaagtt tatatatttt tttaaatttc tgttcaataa taaattaacc ttactttact 840
gtaacttttt aaacttttta atttttccta acattttgac ttttgtaata cagcttaaaa 900

cacacattat acagctatac aaatttttct ttccttatat ctttattctg taagcttttt 960
tccatattta aaattttttg tttgttttta cttattaaac ttttttgtta aaaactaaga 1020
catgcatgca cattaaccta ggcctacaca gggtcaggac catcaatatc attgtcttcc 1080
acttccacat cttgtcccac tggaagatct tcaggggcag taacacacgt ggagctgtca 1140
tctcctataa taacattgcc ttcttttgga atacctcctg aaggacctat ccaaggctgt 1200
ttatagttaa cttttttttt tttttttttt tttttttagt aaataggagg agtacactat 1260
aaaataacaa tataggtgct ataccattat acaactgaca gtgcagtagg tttgtttaca 1320
ccagcatcac cacaaacacg tgagcaatgt gtcgtactac agtgttagga tggctataac 1380
atcactaagc aataggaact tttaaactcc attataatct tatgggacca ctatcacata 1440
tgcaatctcc tgtggaccaa aatgtcatta tgtggtacat gactgtacta agaaattgat 1500
ccatctatat tccatcaatt tgtttagggc tttttctggt tacatttacc tgtgagccca 1560
gaaaaccagt tttgtagaaa ttaacttctg taatgctagg agttaaaaaa aattgctgaa 1620
caacttttac attgttaaac atttaaaaac aagcgttcta gaagtttatc aaatttcata 1680
aaggtgcaaa aatgtaaatg taaatcatta tccagctaat atatatgttg tatttcccta 1740
gtaggagagc atatgtacct cttcctagtt atacaaattt gatatatagt aaagaaacag 1800
taaattctac ttcaagtcat tttgggagga ttaaaaactg aatttctcta gtttgaccat 1860
tgtacagatt tatctggcaa ttttactaaa acctgattta taggttaaac ttggtgtata 1920
tcatatatca ctttacttta gaggaattaa gatttcacat aaatccattt ccaggttcca 1980
aagaccagga agaggcttgg tttttgtttt tctttttact gtctttacag tctccttgac 2040
ttttcttagg agagaaggta ctgagaaaac atgattctaa tatttattat tttttcttcc 2100
aacattttct tatgaaacat tttcaaatac aaaattgagt tttatttaaa acatttgcaa 2160
atatactacc tagattctac cattgttgtt ttatatttgc tttacttaca acttttaaaa 2220
gatgcttttt ataccactga acattttagc ttacatttca caaagaaaag aaaaaattta 2280
agagactttg cataatgttt taagggggttg cagtaaagaa gtgcttctta tatttcttta 2340
tgcatacaaa tcagctgggc ttattaaaat ccagattcta attcagaagg tttaggtggg 2400
gaccgagtct gcatttctaa caaactccta ggtggtattt ttcttggtac ttggaccata 2460
ctttgagtag aaaagcagta gaggacataa aaagagtctt gttagtccca ctttgttgct 2520
gtccacttct catttgataa tatcctaaaa tagctgtgtc tcctttttgg tggttgtatg 2580
attactacct cagaagtact aattgattct tgctatttga ccttaatact ttaatataac 2640
acagcattca tatttgatca gaaaactatc tggcttcctt ttataagaga tttttaggtt 2700
ttatacagtt ttgtggcctt gggttttttt gtttgatttg tttttttgaa ggtatataat 2760
atgtaagtag ataaacaaat ttgatttgta gacattttta tgtggatcat ctaattaaaa 2820
atggagggat acagtatgaa agaatacttg tacttcttaa cagagcactc aacctttctt 2880
ttacatcctg tttcactgat gttattatgt aatttatgtt gctaaactat aaattagata 2940
tttaatttct gttctttgat ttccttttat tattaaatgg acttgttgat ttgcctagaa 3000
attaatttgc ctttcaaaag tcttattaat cttcctccgt tgaaattaat ttgatatttg 3060
catgcttctg gaagacttta aagagctatt ccgagtaact gtagagatta taaaatgaaa 3120
tatgggaatt ttaataaatt ttacatctcc agttactggt gaaaatgtca agtcctcctt 3180
tctgcagagt attttgttac tcatctgtta ttcagcttat ttatttattt atttatttat 3240
ttatttttct ttctttcttg tttttttttt ttgagacgga gtcttgcttt gtcgcccagg 3300
ctggagtaca gtggtgggat cttggctcac tgcaggctcc gcctcccggg ttcacaccat 3360
tcttctgcct cagcctccca agtagctggg actacaggca cccgccacca tgccttgcta 3420
aatttttgta tttttagtag agacgggttt cactgtgtta gccaggatgg tctcgatctc 3480
ttgacctcgt gatccacctg cctcggcctc ccaaagtgct gggattacag gcatgagcca 3540

<210> 5
<211> 20
<212> DNA
<213> Artificial Sequence
<220>
<223> Description of Artificial Sequence: KRAS forward primer
<400> 5
gaatggtcct gcaccagtaa 20

<210> 6
<211> 24
<212> DNA
<213> Artificial Sequence
<220>
<223> Description of Artificial Sequence: KRAS reverse primer
<400> 6
gtgtgacatg ttctaatata gtca 24

## Description

### TECHNICAL FIELD

The present invention relates to a method for predicting sensitivity of a subject to an EGFR inhibitor through checking the genotype of a KRAS protein in a biological sample which has been minimally invasively collected from the subject.

Priority is claimed on Japanese Patent Application No. 2013-057033, filed on March 19, 2013, the content of which is incorporated herein by reference.

### BACKGROUND ART

An epidermal growth factor receptor (EGFR) is a member of the ErbB family of extremely well associated receptors which includes EGFR (ErbB-1), Her2/neu (ErbB-2), Her3 (ErbB-3), and Her4 (ErbB-4). The ErbB family is the type 1 tyrosine kinase family of a growth factor receptor which plays an important role in growth, differentiation, and survival of cells. Activation of these receptors typically occurs through specific ligand bonding and forms a hetero- or homo-dimer between the receptor family members. Subsequently, this causes autophosphorylation of a tyrosine kinase domain.

The activation of EGFR derives a series of signaling events in a downstream of EGFR which mediate between activation of receptor tyrosine kinase, cell growth, cell motility, cell adhesion, cellular infiltration, and a tolerance to chemotherapy, in addition to inhibition of apoptosis which is an important process for continuous growth or survival of cancer cells.

In addition, this family member including EGFR and Her2 is directly related to cellular transformation. For this reason, a molecular target drug which targets EGFR has been developed as an anti cancer agent. Clinical tests of two primary types of EGFR inhibitors of an anti-EGFR antibody and a low molecular EGFR tyrosine kinase inhibitor (TKI) have been carried out so far. An anti-EGFR antibody such as cetuximab is designed so as to bind to an extracellular domain of EGFR and to block the activation of signaling in the downstream of EGFR. Cetuximab (which has been known as antibody 225; refer to Patent Document 1) is prepared with respect to an A431 cell expressing high-level wild type EGFR. In contrast, low molecular KTI such as gefitinib (compound ZD1839; Iressa) or erlotinib (compound OSI-774; Tarceva) competes withATP for bonding to an intracellular catalytic domain of EGFR tyrosine kinase. As a result, EGFR autophosphorylation and signaling of the downstream of EGFR are inhibited.

In recent years, the relationship between therapeutic efficacy of panitumumab or cetuximab, which is a molecular target drug of colorectal cancer and a mutated KRAS gene or a protein thereof, the relationship between therapeutic efficacy of erlotinib or gefitinib which is a molecular target drug of lung cancer, and a mutated EGFR gene or a protein thereof, and the relationship between therapeutic efficacy of crizotinib which is an ALK inhibitor, and a fusion gene accompanied by translocation or a protein thereof have become clinically clear. In use of these drugs, companion diagnosis including examination of mutation of KRAS has been attracting attention. Specifically, among patients with phase III colorectal cancer in a CRYSTAL (FOLFIRI) test, the efficacy during combined use of cetuximab for patients with mutated-KRAS tumors is 36.2% whereas the efficacy during combined use of cetuximab for patients with wild type KRAS tumors is 59.3%, which shows a result that the efficacy of cetuximab with respect to patients with mutated-KRAS tumors is drastically low (for example, refer to Non Patent Document 1). Thereafter, even for a patient with phase II colorectal cancer in an OPUS (FOLFOX) test, it has been proved that cetuximab is efficient for patients with mutated-KRAS tumors whereas the efficacy of cetuximab for patients with mutated-KRAS tumors is extremely low. From these clinical test results, it has been determined that a mutated KRAS gene in a patient with colorectal cancer becomes a predictive factor for therapeutic effect of an EGFR inhibitor (for example, refer to Non Patent Document 2). In a case where therapeutic results are poor even in the KRAS wild type group, clinical test results in which mutation is present in BRAF or PIK3CA have been reported. However, currently, there is not enough evidence as a super non-responder which transcends KRAS

When performing EGFR inhibitor therapy through diagnosis of KRAS gene, there are three clinical problems as follows which relate to sampling from a subject and to a drug tolerance. A first problem is that a biopsy specimen is used for diagnosis of KRAS gene in a case in which surgical resection is not performed, but its reliability has not been confirmed. In addition, acquisition of biological tissues is invasive for a patient, and there are many cases in which a recurrent tumor cannot be re-resected after resection of a primary lesion. For this reason, a diagnostic marker with high reliability which can be replaced with method for directly checking the status (genotype of KRAS) of KRAS in a tumor tissue has been expected.

A second problem is that mutation statuses are not necessarily coincident with each other between a metastatic lesion and a primary lesion.

Watanabe et al., reported that 15 cases out of 43 cases of patients with colorectal cancer are patients with mutated-KRAS tumors and the coincidence rate between the primary lesion and the metastatic lesion is 88.4% (refer to Non Patent Document 3). That is, about 10 percent of the KRAS mutation statuses are not coincident with each other between the primary lesion and the metastatic lesion. In contrast, S. Gattenlohner et al., reported a change between a primary lesion and a metastatic lesion during EGFR inhibitor therapy. As a result of examination of the statuses before and after therapy of 21 cases of metastatic colorectal cancer, there is no change in 20 cases (95.2%). There is heterogeneity in 1 case, in which there is a change, and the case includes multiple cases (refer to Non Patent Document 4).

A third problem is that there is a possibility that the number of mutant clones increases during EGFR inhibitor therapy, that is, acquisition of a tolerance with respect to an EGFR inhibitor. Diaz Jr. et al. detected a DNA-mutated KRAS gene in blood plasma after starting EGFR inhibitor therapy among 24 cases in which a primary lesion was wild type colorectal cancer. As a result, it has been reported that a mutated KRAS gene was detected from 9 cases (37.5%), mutation was observed in all of the cases at least up to the 26th week after administration, a tolerance was acquired at the same time as the detection of the mutation of the extracellular DNA, or after observation. Furthermore, they have suggested that it is possible to estimate acquisition of a tolerance of an EGFR inhibitor by detecting a mutated KRAS gene in serum with high sensitivity, for example, it is possible to switch the EGFR inhibitor to another drug such as MEK inhibitor (refer to Non Patent Document 5). In addition, Misale et al. also suggested to predict sensitivity with respect to an EGFR inhibitor by detecting KRAS gene mutation in blood plasma with high sensitivity and to switch the EGFR inhibitor to another drug in a case where KRAS gene mutation is detected in blood plasma (refer to Non Patent Document 6). That is, in Non Patent Document 5 and 6, there is a disclosure that the status of KRAS in blood is identified using the fact that KRAS existing in a metastatic lesion of a subject is detected in circulating DNA as well, and accordingly, it is possible to predict efficacy when administering an EGFR inhibitor to the subject after checking sensitivity with respect to the EGFR inhibitor of the metastatic lesion.

In contrast, clinical tests relating to re-administration of an EGFR inhibitor have been carried out as a solution to overcome a drug tolerance of cancer. Significant extension of progression-free survival (PFS) has been recognized by re-administering the EGFR inhibitor in comparison with a case in which the EGFR inhibitor is not re-administered. It is considered that this is because mutant clones grow during EGFR inhibitor therapy and a tumor acquires a tolerance with respect to the EGFR inhibitor, but the number of mutant clones relatively decreases by switching the therapy to another therapy, and when the EGFR inhibitor is re-administered at this time, the EGFR inhibitor becomes effective again. Actually, a re-challenging prospective test has been carried out by performing therapy again using cetuximab after performing another therapy for a certain period at a point in time at which the efficacy of cetuximab with respect to a tumor of a human patient has decreased, and the ongoing process thereof has been reported by Santini et al. (refer to Non Patent Document 7). In the report, cetuximab is administered during 1 st line therapy of patients with wild type KRAS tumors which is then switched to another anti cancer therapy (for example, chemotherapy such as XELOX or FOLFOX) at a point in time at which the efficacy has decreased, and cetuximab is further administered again after the lapse of a certain period of time. In this prospective test, PFS has been extended only in the case of the KRAS wild type patient, and a life prolongation effect has been confirmed.

### PRIOR ART DOCUMENTS

### PATENT DOCUMENTS

[Patent Document 1] United States Patent No. 4,943,533

### NON PATENT DOCUMENTS

[Non Patent Document 1] Cutsem et al., The New England Journal of Medicine, 2009, vol. 360, pp. 1408-1417
[Non Patent Document 2] Bokemeyer et al., Annals of Oncology, 2011, vol. 22(7), pp. 1535-1546
[Non Patent Document 3] Watanabe et al., Diseases of the Colon and Rect, 2011, vol. 54, pp. 1170-1178
[Non Patent Document 4] Gattenlohner et al., New England Journal of Medicine, 2009, vol. 360(8), p.835
[Non Patent Document 5] Diaz Jr. et al., Nature, 2012, vol. 486, pp.537-540
[Non Patent Document 6] Misale et al., Nature, 2012, vol. 486(7404), pp. 532-536
[Non Patent Document 7] Santini et al., Annals of Oncology, 2012, vol. 23, pp. 2313-2318

### DISCLOSURE OF INVENTION

### PROBLEMS TO BE SOLVED BY THE INVENTION

The present invention relates to a method for predicting sensitivity of a patient with an EGFR-mediated tumor (cancer) to an EGFR inhibitor using the status of KRAS in peripheral blood of the patient as an indicator regardless of the status of KRAS in a tumor tissue of the patient.

### MEANS FOR SOLVING THE PROBLEMS

The present inventors have conducted extensive studies in order to solve the above-described problems. As a result, they have completed the present invention by finding that, there is a case where the status of KRAS in a tumor tissue and the status of KRAS in peripheral blood are not coincident with each other in a patient with an EGFR-mediated tumor (cancer), and the efficacy of an EGFR inhibitor is low in a patient in whom mutant KRAS is detected in peripheral blood while mutant KRAS is not detected in a tumor tissue.

A method for predicting sensitivity to an EGFR inhibitor according to a first aspect of the present invention, includes: (a) determining whether there is a KRAS gene-derived nucleic acid or a protein thereof in a blood sample which has been collected from a subject, and whether the KRAS gene-derived nucleic acid or the protein thereof in the blood sample is wild type or mutant; and (b) determining that there is a high possibility that a tumor of the subject is sensitive to an EGFR inhibitor when a wild type KRAS gene-derived nucleic acid or a protein thereof is detected and no mutant KRAS gene-derived nucleic acid or a protein thereof is detected in the blood sample in the process (a), and determining that there is a high possibility that the tumor of the subject is not sensitive to the EGFR inhibitor in a case where a mutant KRAS gene-derived nucleic acid or the protein thereof is detected in the blood sample.

In the first aspect, even if a genotype of a KRAS gene-derived nucleic acid or a protein thereof which is detected from a tissue specimen or a cell specimen which has been collected from the tumor of the subject is different from a genotype of the KRAS gene-derived nucleic acid or the protein thereof which is detected from the blood sample, it may be determined that there is a high possibility that the tumor of the subject is not sensitive to the EGFR inhibitor in a case where the mutant KRAS gene-derived nucleic acid or the protein thereof is detected in the blood sample which has been collected from the subject, in the process (b).

In the first aspect, the subject may have received surgical resection treatment which had been performed on a tumor site in the past.

In the first aspect, the subject may have been administered with the EGFR inhibitor in the past.

In the first aspect, the subject may have exhibited a drug tolerance to the EGFR inhibitor in the past.

In the first aspect, the blood sample may be collected from a subject 60 days after administration of the EGFR inhibitor.

In the first aspect, the subject may be a tumor patient who has received an antitumor therapy which is different from an EGFR inhibitor administration treatment after reception of the EGFR inhibitor administration treatment, and the blood sample may be collected before the tumor patient receives the EGFR inhibitor administration treatment again.

In the first aspect, the antitumor therapy which is different from the EGFR inhibitor administration treatment may be therapy of administering a chemotherapeutic agent.

In the first aspect, the chemotherapeutic agent may be one or more selected from the group consisting of fluorouracil, folinic acid, oxaliplatin, irinotecan, cytarabine, fludarabine, gemcitabine, hydroxyurea, methotrexate, bleomycin, chlorambucil, cisplatin, cyclophosphamide, doxorubicin, mitoxantrone, camptothecin, topotecan, teniposide, colcemid, colchicine, paclitaxel, vinblastine, vincristine, and tamoxifen.

In the first aspect, the antitumor therapy which is different from the EGFR inhibitor administration treatment may be a radiation therapy.

In the first aspect, the antitumor therapy which is different from the EGFR inhibitor administration treatment may be a therapy of administering a molecular target drug which is different kind from the EGFR inhibitor that has already been administered to the subject.

In the first aspect, the molecular target drug may be one or more selected from the group consisting of cetuximab, panitumumab, bevacizumab, gefitinib, erlotinib, regorafenib, crizotinib, sunitinib, sorafenib, everolimus, trastuzumab, lapatinib, and rituximab.

In the first aspect, the antitumor therapy which is different from the EGFR inhibitor administration treatment may be a combined therapy of the therapy of administering the molecular target drug and the therapy of administering the chemotherapeutic agent.

In the first aspect, the tumor may be a recurrent tumor.

In the first aspect, the tumor may be a metastatic lesion.

In the first aspect, the tumor may be a primary lesion.

In the first aspect, the tumor may be one or more selected from the group consisting of colorectal cancer, colon cancer, rectal cancer, lung cancer, liver cancer, breast cancer, ovarian cancer, prostate cancer, kidney cancer, esophageal cancer, head and neck cancer, uterine cancer, and cervical cancer.

In the first aspect, the tumor may exist in a plurality of sites in a body of the subject.

In the first aspect, the mutant may be one or more selected from the group consisting of G12A, G12C, G12D, G12R, G12S, G12V, G13D, G12S2, G13A, G13S, G13V, G13R, G13C, Q61H, Q61L, Q61R, A146T, andA146V of a KRAS protein.

In the first aspect, the determination of the presence and absence of the KRAS gene-derived nucleic acid or the protein thereof in the blood sample and the determination whether the KRAS gene-derived nucleic acid or the protein thereof is wild type or mutant may be performed by checking whether the wild type KRAS gene-derived nucleic acid is detected or the mutant KRAS gene-derived nucleic acid is detected from a circulating DNA in the blood sample.

In the first aspect, the blood sample may be one of the group consisting of peripheral blood, serum, and blood plasma.

In the first aspect, CEA in the blood sample may be less than or equal to 5 ng/mL or the CA19-9 value in the blood sample may be less than or equal to 37.0 U/mL.

### Effects of the Invention

According to the method for predicting sensitivity to an EGFR inhibitor of the above-described mode, it is possible to accurately predict sensitivity of a subject to an EGFR inhibitor from a biological sample less invasively obtained from the subject.

### EMBODIMENTS FOR CARRYING OUT THE INVENTION

A method for predicting sensitivity to an EGFR inhibitor according to an embodiment of the present invention (hereinafter, in some cases, referred to as "method for predicting sensitivity according to the present invention") is characterized in that the sensitivity to an EGFR inhibitor is predicted using the status of KRAS in blood as an indicator regardless of the status of KRAS in a tumor tissue.

If a subject whose KRAS in blood is a wild type and in whom no mutant type has been detected in the blood, it is predicted that the sensitivity to an EGFR inhibitor would be high regardless of whether KRAS in a tumor tissue is a wild type or a mutant type. In contrast, if a subject in whom mutant type KRAS has been detected from blood, it is predicted that the sensitivity to an EGFR inhibitor would be low regardless of whether KRAS in a tumor tissue is a wild type or a mutant type.

In the related art, it has been known that the EGFR inhibitor is not effective if there is a mutated KRAS gene or a protein thereof (mutated KRAS gene-derived protein) in a tumor tissue. Furthermore, it has been known that mRNA of a KRAS gene or a KRAS protein is detected also from blood as well as from a tumor tissue of a patient with an EGFR-mediated tumor (cancer), but it has been considered that the status of KRAS in blood and the status of KRAS in a tumor tissue are coincident with each other. However, in some cases, the status of KRAS in blood and the status of KRAS in a tumor tissue are not coincident with each other unlike in the knowledge of the related art. In this case, unexpectedly, the efficacy of an EGFR inhibitor on a tumor tissue is based on the status of KRAS in blood rather than on the status of KRAS in a tumor tissue.

Actually, as shown in Example 1 to be described below, even though no mutated KRAS gene has been observed in both a primary lesion and a metastatic lesion in the analysis of clinical results with respect to a patient with recurrent colorectal cancer, tumor reduction effect caused by cetuximab which is an EGFR inhibitor has not been observed in a patient with recurrent colorectal cancer in whom mutant KRAS gene-derived nucleic acid has been detected from circulating DNA in blood. In addition, a tumor of a patient with recurrent colorectal cancer in whom mutant KRAS has been detected in blood does not respond to an EGFR inhibitor even though mutant KRAS has not been detected either in a primary lesion or in a metastatic lesion of a tumor tissue.

That is, even if the presence of a mutated KRAS gene is not recognized in a tumor tissue, it is possible to predict that the subject in whom a mutated KRAS gene is detected from circulating DNA in peripheral blood would have low sensitivity to an EGFR inhibitor and low efficacy of the EGFR inhibitor. Similarly, even if the presence of a mutated KRAS gene is not recognized in a tumor tissue of a primary lesion and a metastatic lesion, in a case where the presence of a mutated KRAS gene is identified from circulating DNA in peripheral blood, it is possible to predict that the tumor would be a tumor which does not response to an EGFR inhibitor and re-administration of the EGFR inhibitor would not be effective. The fact that it is possible to accurately predict sensitivity to an EGFR inhibitor when using the status of KRAS in blood as an indicator in this manner rather than the status of KRAS in a tumor tissue was first discovered by the present inventors.

That is, the method for predicting sensitivity according to the present invention is a method for predicting sensitivity of a subject to an EGFR inhibitor has the following processes (a) and (b):
(a) determining whether there is a KRAS gene-derived nucleic acid or a protein thereof in a blood sample which has been collected from a subject, and whether the KRAS in the blood sample is wild type or mutant; and
(b) determining that there is a high possibility that a tumor of the subject is sensitive to an EGFR inhibitor when a wild type KRAS gene-derived nucleic acid or a protein thereof is detected and no mutant KRAS gene-derived nucleic acid or a protein thereof is detected in the blood sample in the process (a), and determining that there is a high possibility that the tumor of the subject is not sensitive to the EGFR inhibitor in a case where a mutant KRAS gene-derived nucleic acid or the protein thereof is detected in the blood sample.

The mutant KRAS detected through the method for predicting sensitivity according to the present invention includes, for example, all of the forms of mutations such as insertion mutation, inversion mutation, deletion mutation, and point mutation. The KRAS gene which has been mutated in these manners is different from wild type KRAS, which is found in one allele (heterozygosity) or both alleles (homozygosity), and is mutant KRAS which can be found in a somatic line or a germ line. The somatic mutation is caused only in certain kinds of tissues such as, for example, in a tumor tissue, and is not inherited by the germ cell line. The germ line mutation can be found in an arbitrary body tissue.

As the mutant KRAS detected through the method for predicting sensitivity according to the present invention, KRAS which has been mutated involving one or more amino acid substitutions among codons 12, 13, 61, and 146 on exons 2 to 4 of the KRAS gene. Specific examples thereof include a mutant KRAS having one or more mutations which are selected from the group consisting of G12A, G12C, G12D, G12R, G12S, G12V, G13D, G13A, G12S2, G13S, G13V, G13R, G13C, Q61H, Q61L, Q61R, A146T, and A146V of a KRAS protein. The mode each mutant nucleic acid (gene) mutation is shown in Table 1. In addition, an amino acid sequence of KRAS is shown by SEQ ID No: 1, a gene sequence including a codon 12 on an exon 2 of KRAS is shown by SEQ ID No: 2, a gene sequence including a codon 61 on an exon 3 of KRAS is shown by SEQ ID No: 3, and a gene sequence including a codon 146 on an exon 4 of KRAS is shown by SEQ ID No: 4.

**[Table 1]**

| Amino acid substitution | Nucleic acid mutation | Exon |
|---|---|---|
| G12A | 5571G > C | 2 |
| G12C | 5570G > T | 2 |
| G12D | 5571G > A | 2 |
| G12R | 5570G > C | 2 |
| G12S | 5570G > A | 2 |
| G12V | 5571G > T | 2 |
| G12S2 | 5570G > T, 5570G > C | 2 |
| G13D | 5574G > A | 2 |
| G13A | 5574G > C | 2 |
| G13V | 5574G > T | 2 |
| G13R | 5573G > C | 2 |
| G13S | 5573G > A | 2 |
| G13C | 5573G > T | 2 |
| Q61H | 23579A > C | 3 |
| Q61L | 23578A > T | 3 |
| Q61R | 23578A > G | 3 |
| A146T | 25293G > A | 4 |
| A146V | 25294C > T | 4 |

In the method for predicting sensitivity according to the present invention, in the process (a), the status of KRAS in a blood sample is checked instead of checking the status of KRAS in a tumor tissue. The blood sample may be peripheral blood, or may be serum or blood plasma. The blood sample can be less invasively collected from a subject compared to a tumor tissue. Therefore, it is possible to predict sensitivity to an EGFR inhibitor of a subject, such as a patient with a recurrent tumor, from whom it is difficult to collect a biological sample of a tumor tissue. In addition, the blood sample can be collected from a subject with time. Therefore, the method for predicting sensitivity according to the present invention is also suitable for monitoring whether the tumor recurs or not.

The blood sample is preferably collected from a subject in an initial stage of a tumor regardless of a primary tumor, a metastatic tumor, or a recurrent tumor. Specifically, in the blood sample used in the method for predicting sensitivity according to the present invention, the value of CEA is preferably less than or equal to 5 ng/mL, or the value of CA 19-9 is preferably less than or equal to 37.0 U/mL.

In the method for predicting sensitivity according to the present invention, the EGFR inhibitor to which sensitivity is predicted is not particularly limited as long as the EGFR inhibitor is a substance having an EGFR-inhibiting action in an animal starting with a human, and the EGFR inhibitor may be an anti-EGFR antibody or TKI. Specific examples of the anti-EGFR antibody include cetuximab (product name: Erbitutux (registered trademark), Imclone Systems Inc.) and panitumumab (product name: ABX-EGF, Abgenix, Inc). In addition, examples of the TKI include low molecules, for example, erlotinib (product name: Tarceva) (registered trademark), (OSI Pharmaceuticals, Inc.); gefitinib (product name: Iressa (registered trademark), AstraZeneca); tyrphostins disclosed in Dvir et al., Journal of Cell Biology, vol. 113, pp. 857-865 (1991); tricyclic pyrimidinic compounds disclosed in United States Patent No. 5679683; and compound 6-(2,6-dichlorophenyl)-2-(4-(2-diethylamino)phenylamino)-8-methyl -8H-pyrido(2,3-d) pyrimidin-7-one (which has been known as PD166285) disclosed in Panek et al., Journal of Pharmacology and Experimentral Therapeutics, vol. 283, pp. 1433-1444 (1997), which compete with ATP. In the method for predicting sensitivity according to the present invention, it is preferable to predict sensitivity to one type or more types of these EGFR inhibitors. Among these, it is preferable to predict sensitivity to cetuximab or panitumumab.

In the method for predicting sensitivity according to the present invention, the tumor which is subjected to predict sensitivity to an EGFR inhibitor is not particularly limited as long as the tumor is an EGFR-mediated tumor (cancer), that is, a tumor in which EGFR plays a certain role for forming a tumor. The tumor includes cancer in the brain, the liver, the kidneys, the bladder, the breast, the stomach, the ovary, the colorectum, the prostate, the pancreas, the lung, the vulva, the thyroid, and the esophagus, liver cancer, sarcoma; gliosarcoma; head and neck cancer, leukemia; and lymphoid malignancies. More specifically, the tumor includes neuroblastoma, intestinal cancer (for example, rectal cancer, colorectal cancer, familial polyposis coli cancer, and hereditary nonpolyposis colorectal cancer), esophageal cancer, lip cancer, laryngeal cancer, hypopharyngeal cancer, lingual cancer, salivary gland cancer, gastric cancer, adenocarcinoma, medullary thyroid cancer, thyroid artery papillary cancer, kidney cancer, granular cell carcinoma, ovarian cancer, cervical cancer, uterine cancer, endometrial cancer, choriocarcinoma, pancreatic cancer, prostate cancer, testicular cancer, breast cancer, ureter cancer melanoma, brain tumor (for example, glioblastoma, astrocytoma, meningioma, medulloblastoma, and peripheral neuroectodermal tumor), Hodgkin's lymphoma, non-Hodgkin's lymphoma, Burkitt's lymphoma, acute lymphocytic leukemia (ALL), chronic lymphocytic leukemia (CLL), acute myeloid leukemia (AML), chronic myeloid leukemia (CML), adult T-cell leukemia, hepatoma, gallbladder cancer, bronchial cancer, small cell lung cancer, non-small cell lung cancer, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma, and plasmacytoma. As the tumor which becomes a prediction subject in the method for predicting sensitivity according to the present invention, one type or more types selected from the group consisting of colorectal cancer, colon cancer, rectal cancer, lung cancer, liver cancer, breast cancer, ovarian cancer, prostate cancer, kidney cancer, esophageal cancer, head and neck cancer, uterine cancer, and cervical cancer are preferable.

In the method for predicting sensitivity according to the present invention, the tumor which is subjected to predict sensitivity to an EGFR inhibitor may be a primary lesion (primary tumor) or a metastatic lesion (metastatic tumor).

In addition, the tumor may be recurrent tumor. Furthermore, the tumor may be present in a plurality of sites in the body of a subject.

In the method for predicting sensitivity according to the present invention, it is preferable to predict sensitivity of an EGFR inhibitor with respect to a recurrent tumor. For example, it is possible to predict sensitivity to an EGFR inhibitor of a recurrent tumor or metastatic lesion using a blood sample which has been collected from a subject who had received surgical resection treatment which had been performed on a tumor site in the past or a subject who had been administered with an EGFR inhibitor in the past. In a case where a subject had been administered with an EGFR inhibitor in the past, it is preferable to use a blood sample which has been collected 60 days after administration of the EGFR inhibitor. It is preferable to continuously carry out the method for predicting sensitivity according to the present invention in order to perform therapy while predicting sensitivity to the EGFR inhibitor during the period of EGFR inhibitor therapy. In general, it is considered that it is preferable to carry out the method for predicting sensitivity according to the present invention for about every 60 days in terms of a balance between collection of blood for tumor marker examination to be performed about once a month and a CT examination to be performed about once every three months.

In addition, the blood sample used in the method for predicting sensitivity according to the present invention may be collected from a subject who had exhibited a drug tolerance to an EGFR inhibitor in the past. Even in the subject who had exhibited a drug tolerance to an EGFR inhibitor in the past, in a case where no mutant KRAS gene-derived nucleic acid or a protein thereof is detected from the blood sample, it is possible to determine that the subject is sensitive to the EGFR inhibitor at a point in time at which the blood sample is collected. For this reason, it is possible to determine that there is a high possibility that tumor reduction effect may be obtained by taking the EGFR inhibitor. In contrast, in a case where a mutant KRAS gene-derived nucleic acid or a protein thereof is detected in the blood sample, it is possible to determine that the subject is less sensitive to the EGFR inhibitor, and there is a high possibility that tumor reduction effect may not be obtained even if and the subject takes an EGFR inhibitor, at a point in time when the blood sample of the subject has been collected.

The method for predicting sensitivity according to the present invention is preferably performed on a blood sample which has been collected from a subject who re-challenges the EGFR inhibitor. Here, the "subject who re-challenges an EGFR inhibitor" means a subject who has received a second antitumor therapy which is different from EGFR inhibitor administration treatment after reception of the EGFR inhibitor administration treatment, and then, may receive the EGFR inhibitor administration treatment again. In some cases, a tolerance is acquired through administration of an EGFR inhibitor. However, it is possible to predict the efficacy of the EGFR inhibitor during re-challenging of the EGFR inhibitor by checking the status of KRAS in blood in advance before the re-challenging of the EGFR inhibitor. That is, in a case where no mutant KRAS gene-derived nucleic acid or a protein thereof is detected from a blood sample which has been collected from a subject before re-challenging the EGFR inhibitor, it is possible to determine that there is a high possibility that the subject may be sensitive to the EGFR inhibitor and tumor reduction effect may be obtained by re-challenging the EGFR inhibitor. In contrast, in a case where the mutant KRAS gene-derived nucleic acid or the protein thereof is detected in the blood sample, it is possible to determine that there is a high possibility that the subject is less sensitive to the EGFR inhibitor and tumor reduction effect cannot be obtained even if the EGFR inhibitor is re-challenged.

As the second antitumor therapy to be received before re-challenging the EGFR inhibitor, it is possible to appropriately select therapy from well-known antitumor therapy depending on the clinical condition of a subject. Specific examples of the second antitumor therapy include radiation therapy, therapy of administering a chemotherapeutic agent, and therapy of administering molecular target drug which is different in type from the EGFR inhibitor which has already been administered. As the second antitumor therapy, one or more types of the antitumor therapy may be used in combination. For example, as the method for predicting sensitivity according to the present invention, it is preferable to use the therapy of administering a molecular target drug and therapy of administering a chemotherapeutic agent in combination.

The chemotherapeutic agent is not limited and can be a compound having cytotoxicity or cell division inhibitory properties. Specific examples thereof include (i) antimetabolites, for example, fluorouracil, cytarabine, fludarabine, 5-fluoro-2'-deoxyuridine, gemcitabine, hydroxyurea, or methotrexate; (ii) DNA fragmentation agents, for example, bleomycin; (iii) DNA cross-linking agents, for example, chlorambucil, cisplatin, cyclophosphamide, or nitrogen mustard; (iv) intercalating agents, for example, adriamycin (doxorubicin) or mitoxantrone; (v) protein synthesis inhibitors, for example, L-asparaginase, cycloheximide, puromycin, or diphtheria toxin; (vi) topoisomerase I poisons, for example, camptothecin, or topotecan; (vii) topoisomerase II poisons, for example, etoposide (VP-16) or teniposide; (viii) microtubule-associated agents, for example, colcemid, colchicine, paclitaxel, vinblastine, or vincristine; (ix) kinase inhibitors, for example, flavopiridol, staurosporine, STI571 (CPG57148B), or UCN-01 (7- hydroxystaurosporine); (x) various investigational drugs, for example, thioplatin, PS-341, phenylbutyrate, ET-18-OCH3, or farnesyltransferase inhibitors (L-739749 and L-744832); polyphenols, for example, quercetin, resveratrol, piceatannol, epigallocatechin gallate, theaflavins, flavanols, procyanidins, betulinic acid, and derivatives thereof; (xi) hormones, for example, glucocorticoid, or fenretinide; (xii) anti-hormones, for example, tamoxifen, finasteride, or LHRH antagonist. In addition, folinic acid, oxaliplatin, irinotecan, daunorubicin, taxotere, and mitomycin C are included therein. In the second antitumor therapy, only one kind among these chemotherapeutic agents may be used or two or more kinds thereof may be used in combination.

In a case where the method for predicting sensitivity according to the present invention is performed on a blood sample which has been collected from a subject who has been scheduled to further re-challenge an EGFR inhibitor after performing therapy of administering a chemotherapeutic agent after treatment of administering the EGFR inhibitor, one or more kinds selected from the group consisting of fluorouracil, folinic acid, oxaliplatin, irinotecan, cytarabine, fludarabine, gemcitabine, hydroxyurea, methotrexate, bleomycin, chlorambucil, cisplatin, cyclophosphamide, doxorubicin, mitoxantrone, camptothecin, topotecan, teniposide, colcemid, colchicine, paclitaxel, vinblastine, vincristine, and tamoxifen are preferably used as the chemotherapeutic agents.

Specific examples of the molecular target drug include one or more selected from the group consisting of cetuximab, panitumumab, bevacizumab, gefitinib, erlotinib, regorafenib, crizotinib, sunitinib, sorafenib, everolimus, trastuzumab, lapatinib, and rituximab.

In the step (a), the status of KRAS in a blood sample may be determined by the level of a protein, or by the level of a nucleic acid (genome DNA or mRNA). In the method for predicting sensitivity according to the present invention, it is preferable to set a KRAS gene-derived nucleic acid as a measurement object since it is possible to detect the KRAS gene-derived nucleic acid with high sensitivity. Specifically, it is preferable to perform determination whether there is KRAS in the blood sample and whether the KRAS is a wild type or a mutant by checking whether a wild type or mutant KRAS gene-derived nucleic acid is detected, from circulating DNA in the blood sample. Examples of the KRAS gene-derived nucleic acid include the total length of genome mRNA of a KRAS gene or a part thereof, the total length of genome DNA of a KRAS gene or a part thereof, cDNA obtained by using the total length of the mRNA or a part thereof as a template, or an amplification product in which these genome DNA, mRNA, and cDNA are artificially amplified through polymerase chain reaction (PCR) or the like.

Detection of a KRAS gene-derived nucleic acid in a blood sample or determination of a genotype of the detected KRAS gene-derived nucleic acid can be performed through a usual method.

For example, the existence of KRAS in a blood sample or the status thereof can be determined through detecting a KRAS gene-derived nucleic acid which has been contained in the blood sample using digital PCR. Particularly, it is possible to detect the KRAS gene-derived nucleic acid with high sensitivity using technology (Hindson et al., Analytical Chemistry, 2011, vol. 83 (22), pp. 8604-8610) of droplet digital PCR (ddPCR) of Bio-Rad Laboratories, Inc. The larger the number of droplets is, the higher the analytical accuracy is. In order to secure performance of detecting 0.01% mutation, 10,000 droplets are required for detecting one instance of mutation. For this reason, it is preferable to define the concentration of a surfactant in Master Mix of PCR. For example, it is preferable that the final concentration of ethylene glycol or glycerol which is used as a preservation solution of a DNA extension enzyme or the like is less than or equal to 0.15% or the final concentration of Triton-X is less than or equal to 0.0003%. When the above-described surfactant becomes greater than or equal to the final concentration, the number of emulsions due to the droplets is sharply decreased. Therefore, it is difficult to detect mutation with high sensitivity.

In addition, it is preferable to perform a well-known method for detecting mutation after increasing the absolute amount of the number of copies of allyl of mutant KRAS which may be contained in the nucleic acid by performing 15 cycles to 50 cycles of first PCR using the nucleic acid and a nucleic acid fragment which are obtained from a blood sample, and then, diluting the number of copies thereof to 106. According to the method, the total number of mutant existing in a reaction system is increased. Therefore, even if the type of mutation increases, it is possible to reduce the possibility that a mutant allyl will not be detected since the allyl is not physically present. Furthermore, the method may be combined with the digital PCR.

Examples of other methods include a method of amplifying a fragment including a region which encodes a mutation site in a KRAS gene through PCR or the like in which the nucleic acid in the blood sample is used as a template, and then, detecting whether an assembly is formed by bringing a probe, which is specifically hybridizable with a specific genotype of KRAS, into contact with this amplification product, with high sensitivity. It is preferable to perform emulsion PCR on a diluted product of the amplification product before performing the hybridization.

The probe is labeled so as to be detectable using, for example, a radioactive isotope (³H, ³²P, ³³P, or the like), a fluorescent agent (rhodamine, fluorescein, or the like), or a color former. In addition, the probe may be antisense oligomers, for example, PNA, morpholino phosphoroamidates, or LNA. The base length of the probe may be about 8 nucleotides to about 100 nucleotides, about 10 nucleotides to about 75 nucleotides, about 15 nucleotides to about 50 nucleotides, or about 20 nucleotides to about 30 nucleotides.

The existence of KRAS in a blood sample or the status thereof can be analyzed using an invader (registered trademark) method (Michael Olivier, Mutation Research 573: 103-110, 2005). The invader method is a method in which an allele probe and invader oligo perform hybridization so as to form a partial triple base with respect to double stranded DNA or mRNA which is prepared through PCR or the like. Here, a part of the 5' terminal of the allele probe is designed so as to have a noncomplementary sequence (flap portion) to the double stranded DNA or mRNA. In contrast, the invader oligo has a sequence which is completely complementary thereto. Each of the two kinds of allele probes is designed so as to be complementary to a wild type and a mutant. When hybridization has been completely complementally carried out by performing hybridization competitively with respect to the above-described double stranded DNA or mRNA, a flap endonuclease recognizes a part which becomes a triple base, and the flap portions of the allele probes are hybridized with a self-complementary FRET cassette existing in the reaction system.

At this time, a part which becomes a triple base is generated, the flap endonuclease disconnects target mutation, and fluorescence is emitted since a fluorescence-modified DNA fragment in the FRET cassette is separated from the FRET cassette and is deviated from quenching matter in FRET. Theoretically, the flap portions of the allele probes which once have been disconnected can be hybridized again with other FRET cassettes, and therefore it is possible for this method to amplify a signal and to detect mutation with significantly high sensitivity.

Ligase chain reaction which has been known in the field can be used in order to amplify a fragment including a region encoding a mutation site in a KRAS gene (for example, refer to Wu et al., Genomics, 1989, vol. 4, pp. 560-569). In addition, it is possible to use technology which has been known as allele-specific PCR (for example, refer to Ruano and Kidd, Nucleic Acids Research, 1989, vol. 17, p. 8392). According to the technology, a primer which is hybridized with specific KRAS mutation at the 3' terminal is used. In a case where there is no specific KRAS mutation, no amplification product is observed. In addition, it is also possible to use Amplification Refractory Mutation System (ARMS) (for example, refer to European Patent Application, Publication No. 0332435, and Newton et al., Nucleic Acids Research, 1989, vol. 17, No.7).

In detection of a KRAS gene-derived nucleic acid in a blood sample or determination of the genotype of the detected KRAS gene-derived nucleic acid, it is also possible to use other methods which are used when detecting gene mutation or when detecting insertion and deletion of a gene. Examples of the methods include a method of using a sequence analysis method based on a Sanger's method and directly determining a base sequence of genome DNA or mRNA of a KRAS gene in a blood sample, an amplification product thereof, or the like. In addition, it is possible to perform determination of a base sequence through PCR. In addition, it is possible to use a restriction fragment length polymorphism (RFLP) probe with respect to a gene or a marker gene around the gene in order to score modification or insertion of an allele in a polymorphism fragment. It is also possible to use single strand conformation polymorphism (SSCP) analysis in order to detect a base change mutant of an allele (Orita et.al., Proceedings of the National Academy of Sciences, USA, 1989, vol. 86, pp. 2766-2770, and Genomics, 1989, vol. 5, pp. 874-879).

It is possible to more simply perform the method for predicting sensitivity according to the present invention by making a reagent or the like, which is used in detection of a KRAS gene-derived nucleic acid in a blood sample or in determination of the genotype of the detected KRAS gene-derived nucleic acid, into a kit. Examples of the reagent include a reagent for extracting a nucleic acid from a blood sample; an enzyme such as polymerase or ligase; a probe or a primer (oligonucleotides which is specifically hybridized with a mutation site of a KRAS gene or its adjacent site) which is specifically hybridizable with a specific genotype of KRAS. In addition, the kit may include a document or the like in which a protocol about detection of a KRAS gene-derived nucleic acid in a blood sample or about a method of determining the genotype thereof, or an instruction of a criterion of sensitivity to an EGFR inhibitor from the obtained result of the genotype (status) of KRAS is stated.

The method for predicting sensitivity according to the present invention can provide information which is important when determining whether treatment of administering an EGFR inhibitor is applied. That is, the method for predicting sensitivity according to the present invention can provide useful information to clinicians who can determine an appropriate therapeutic method based on the information obtained through the method.

### Example

Hereinafter, the present invention will be specifically described by showing an example, but is not limited to the following example.

### Example 1

Regarding 23 patients with recurrent colorectal cancer for whom surgical resection of a primary lesion is performed, mutation accompanied by nonsynonymous amino acid substitution was checked about KRAS which was contained in serum which had been prepared from a primary lesion, a metastatic lesion, and blood which had been collected after confirmation of a metastatic lesion.

### Clinical Sample

Before or after surgical resection operation of a primary lesion, a serum component was obtained by performing centrifugal separation processing (for 10 minutes at 3,000 rpm) after collecting 6 mL of peripheral blood of each patient with recurrent colorectal cancer. Furthermore, similarly, a serum component (sample number 16) was obtained by also collecting 6 mL of peripheral blood from a patient with ID number 9 after surgical resection operation of a metastatic lesion. In addition, formalin-fixed paraffin-embedded (FFPE) segments of a primary lesion or a metastatic lesion of some patients were also set to test samples. This test was approved by the Ethical Reviewed Board in Nippon Medical School Hospital and was performed through obtaining the informed consents to include this research from all of the patients. The information of patients in this test is shown in Table 2. In Table 2, "-" in the column of the "metastatic lesion" means a patient in a state of before a metastatic lesion is checked. In addition, in Table 2, "present" in the column of the "cetuximab" means that treatment of administering cetuximab was performed after surgical resection operation of a metastatic lesion (but before surgical resection operation of primary lesion for sample number 8 and sample number 15) and "none" in the same column means that the administration treatment was not performed thereafter. In Table 2, the column of "chemotherapy" indicates the situation of carrying out the chemotherapy at a point in time of treatment of administering cetuximab after surgical resection operation of a metastatic lesion (but at a point in time of treatment of administering cetuximab after surgical resection operation of a primary lesion in sample numbers 20 and 21 and at a point in time of collecting a blood sample in sample number 16). More specifically, "none" means a state in which cetuximab not yet administered and a patient who has a possibility to be administered later. "Before being carried out" means a state in which chemotherapy is scheduled to be carried out. "Being carried out" means a state in which the chemotherapy is effective and the administration is being continued. "After being carried out" means a state in which although the chemotherapy was effective, the administration was completed at the point in time. "After completion" means a state in which the chemotherapy was not effective (that is, a state in which the therapy enters comfort care).

**[Table 2]**

| Sample number | Patient ID | Gender | Age | Primary lesion | Metastatic lesion | Chemotherapy | Cetuximab |
|---|---|---|---|---|---|---|---|
| 1 | 1 | Male | 67 | Ascending colon cancer | Peritoneal metastasis | Being carried out | None |
| 2 | 2 | Male | 70 | Ascending colon cancer | Liver metastasis | After completion | None |
| 3 | 3 | Male | 67 | Lectal cancer | Liver and lung metastasis | Being carried out | None |
| 4 | 4 | Male | 69 | Lectal cancer | Lung metastasis | After completion | None |
| 5 | 5 | Female | 68 | Transverse colon cancer | Peritoneal metastasis | After completion | None |
| 6 | 6 | Female | 50 | Transverse colon cancer | Liver metastasis | Being carried out | Present |
| 7 | 7 | Male | 54 | Lectal cancer | Liver metastasis | Being carried out | Present |
| 8 | 8 | Male | 71 | Lectal cancer | Lung metastasis | Being carried out | Present |
| 9 | 9 | Female | 66 | Lectal cancer | Peritoneal metastasis | None | None |
| 10 | 10 | Male | 78 | Lectal cancer | Liver and lung metastasis | Being carried out | None |
| 11 | 11 | Male | 59 | Lectal cancer | Lung metastasis | Being carried out | None |
| 12 | 12 | Female | 70 | Sigmoid cancer | Liver and lung metastasis | Being carried out | Present |
| 13 | 13 | Female | 79 | Lectal cancer | Liver and lung metastasis | Being carried out | Present |
| 14 | 6 | Female | 50 | Transverse colon cancer | Liver and lung metastasis | Being carried out | Present |
| 15 | 14 | Female | 60 | Lectal cancer | Liver and lung metastasis | Being carried out | Present |
| 16 | 9 | Female | 66 | Lectal cancer | Same as that of patient ID. 9 (no recurrence) | After being carried out | None |
| 17 | 15 | Female | 69 | Lectal cancer | Peritoneal metastasis | None | None |
| 18 | 16 | Male | 66 | Ascending colon cancer | Liver metastasis | Being carried out | Present |
| 19 | 17 | Male | 82 | Lectal cancer | Peritoneal metastasis and lung metastasis | After being carried out | None |
| 20 | 18 | Female | 81 | Ascending colon cancer | - | None | None |
| 21 | 19 | Male | 69 | Lectal cancer | - | Before being carried out | None |
| 22 | 20 | Female | 88 | Lectal cancer | Liver metastasis | None | None |
| 23 | 21 | Female | 81 | Ascending colon cancer | Peritoneal metastasis | None | None |

Anti-EGFR antibody drug therapy was performed on patients with ID number 8 and ID number 15 whose primary lesion was a KRAS wild type. The primary lesion of patients with ID number 8 and ID number 15 were resected after the anti-EGFR antibody drug therapy. Cetuximab chemotherapy and fluorouracil/folinic acid/oxaliplatin (FOLFOX) chemotherapy was performed on the patient with ID number 8. Intravenous administration of cetuximab was performed for over 1 hour at a dose of 780 mg/body through a method for administering cetuximab at 2-week intervals. In the FOLFOX chemotherapy, intravenous administration of folinic acid (leucovorin) at a dose of 300 mg/body and oxaliplatin at a dose of 125 mg/body was performed over 2 hours, and rapid intravenous administration of fluorouracil (5-FU) was performed at a dose of 625 mg/body or 500 mg/body. Then, continuous intravenous administration thereof was performed over 22 hours at a dose of 3800 mg/body. The administration history is shown in Table 3.

**[Table 3]**

| EGFR inhibitor therapy on patient ID. 8 | | | | Unit: mg/body | |
|---|---|---|---|---|---|
| Administration date | Cetuximab | Leucovorin | Oxaliplatin | 5-FU (rapid intravenous injection) | 5-FU (specified continuous intravenous injection) |
| 2012/7/26 | 780 | 300 | 125 | 625 | 3800 |
| 2012/8/9 | 780 | 300 | 125 | 625 | 3800 |
| 2012/8/23 | 780 | 300 | 125 | 625 | 3800 |
| 2012/9/5 | 780 | 300 | 125 | 625 | 3800 |
| 2012/10/17 | 0 | 300 | 125 | 625 | 3800 |
| 2012/11/14 | 0 | 300 | 125 | 500 | 3800 |
| 2012/11/28 | 0 | 300 | 125 | 500 | 3800 |
| 2012/12/12 | 0 | 300 | 125 | 500 | 3800 |
| 2013/1/9 | 0 | 300 | 125 | 500 | 3800 |
| 2013/1/23 | 780 | 300 | 125 | 500 | 3800 |
| 2013/2/6 | 780 | 300 | 125 | 500 | 3800 |
| 2013/2/20 | 780 | 300 | 125 | 500 | 3800 |
| 2013/3/6 | 780 | 300 | 125 | 500 | 3800 |

Panitumumab or cetuximab chemotherapy and fluorouracil/folinic acid/oxaliplatin (FOLFOX) chemotherapy were performed on the patient with ID number 15. Intravenous administration of panitumumab at a dose of 360 mg/body or cetuximab at a dose of 800 mg/body was performed for over 1 hour through a method for administering panitumumab or cetuximab at 2-week intervals. In the FOLFOX chemotherapy, intravenous administration of folinic acid (leucovorin) at a dose of 350 mg/body and oxaliplatin at a dose of 145 mg/body or 140 mg/body was performed over 2 hours, and rapid intravenous administration of fluorouracil (5-FU) was performed at a dose of 675 mg/body or 650 mg/body. Then, continuous intravenous administration thereof was performed over 22 hours at a dose of 4110 mg/body. The administration history is shown in Table 4.

**[Table 4]**

| EGFR inhibitor therapy on patient ID. 15 | | | | | Unit: mg/body |
|---|---|---|---|---|---|
| Administration date | panitumumab | Leucovorin | Oxaliplatin | 5-FU (rapid intravenous injection) | 5-FU (specified continuous intravenous injection) |
| 2012/2/24 | 360 | 350 | 145 | 675 | 4110 |
| 2012/3/8 | 360 | 350 | 145 | 675 | 4110 |
| 2012/3/22 | 360 | 350 | 145 | 675 | 4110 |

| | | | | | Unit: mg/body |
|---|---|---|---|---|---|
| Administration date | Cetuximab | Leucovorin | Oxaliplatin | 5-FU (rapid intravenous injection) | 5-FU (specified continuous intravenous injection) |
| 2012/11/22 | 800 | 350 | 140 | 650 | 4110 |
| 2012/12/5 | 800 | 350 | 140 | 650 | 4110 |
| 2012/12/27 | 800 | 350 | 140 | 650 | 4110 |
| 2013/1/17 | 800 | 350 | 140 | 650 | 4110 |
| 2013/1/31 | 800 | 350 | 140 | 650 | 4110 |
| 2013/2/14 | 800 | 350 | 140 | 650 | 4110 |
| 2013/2/28 | 800 | 350 | 140 | 650 | 4110 |

### Measurement of CEA and CA-19-9 in Serum

CEA and CA-19-9 in serum was measured through chemiluminescence enzyme immunoassay (CLEIA). The measurement results are shown in Table 5.

### Isolation Purification of Cell-free (cf) DNA from Serum

Isolation purification of cfDNA from serum was performed using QIAamp Circulating Nucleic Acid Kit (QIAGEN). The amount of a serum sample which was supplied to this kit varies depending on patients, and was 2 mL to 4 mL. The isolation purification process of DNA was based on instructions attached to the kit. The final elution from a spin column was performed using 50 µL of a TE buffer solution.

### Isolation Purification of DNA from FFPE Segment

DNA isolation purification from a FFPE segment was performed using QIAamp DNA FFPE Tissue Kit (QIAGEN). Three pieces of FFPE segments which were sliced into 10 µm were used for one sample. The isolation purification process of DNA was based on an instruction attached to the kit. The final elution from a spin column was performed using 100 µL of a TE buffer solution.

### DNA Quantitative Determination

Quantitative determination of cfDNA and DNA which was isolated and purified from the FFPE segment was performed using Quant-iT (registered trade name) PicoGreen (registered trade name) dsDNA Reagent and Kits (Invitrogen). All samples to be measured were used by diluting isolated DNA 20 times in a TE buffer solution. SAFIRA (TECAN Group Ltd.) was used as a fluorescence measurement apparatus.

### Direct Sequencing

KRAS base sequence analysis in a surgical specimen of a primary lesion or a metastatic lesion and in serum was performed through direct sequencing. As a primer sequence for direct sequencing of KRAS, KRAS (forward): 5'-GAATGGTCCTGCACCAGTAA-3'(SEQ ID No: 5) and KRAS (reverse): 5'-GTGTGACATGTTCTAATATAGTCA-3' (SEQ ID No: 6) were used. The length of each PCR product was 214 bp. The PCR conditions were as follows: 40 cycles of reactions in which each cycle includes pre-denaturation for 10 minutes at 95°C, 20 seconds at 94°C, 20 seconds at 60°C, and 30 seconds at 72°C; followed by an extension reaction for 10 minutes at 72°C. For the sequence analysis, cycle sequencing through a Big Dye Terminator method was performed using ABI 3730 (Applied Biosystems, Foster City, CA). The results are shown in Table 5. In the column of "serum" in Table 5, "(before operation)" and "(after operation)" in the end of the word of the genotype mean that the genotypes in serum are genotypes in serum which has been collected before or after surgical resection operation of a primary lesion (metastatic lesion only for the case of sample number 16). In addition, "-" in the column of "metastatic lesion" in Table 5 means that the genotype of KRAS in a metastatic lesion has not been analyzed.

**[Table 5]**

| Sample number | Patient ID | Primary lesion | Metastatic lesion | Serum | Amount of serum (mL) | Concentration of cfDNA (ng/µL) | Concentration of CEA (ng/mL) | CA19-9 value (U/mL) |
|---|---|---|---|---|---|---|---|---|
| 1 | 1 | G12v | - | G12V (after operation) | 2 | 0.61 | 7.8 | 8.6 |
| 2 | 2 | G12D | - | G12D (after operation) | 2 | 0.64 | 26.5 | 3726.0 |
| 3 | 3 | G12D | - | G12D (after operation) | 2 | 0.13 | 231.0 | 1991.2 |
| 4 | 4 | G12v | - | wild (after operation) | 2 | 0.49 | 3.5 | 9.2 |
| 5 | 5 | G13D | - | G13D (after operation) | 2 | 0.32 | 3.5 | 3.9 |
| 6 | 6 | wild | - | Q61H (after operation) | 2 | 4.70 | 1234.7 | 2.0 |
| 7 | 7 | wild | - | wild (after operation) | 2 | 3.50 | 2.3 | 11.9 |
| 8 | 8 | wild | wild | G13D (after operation) | 2 | 1.30 | 9.7 | 25.9 |
| 9 | 9 | G12A | - | G12A (before operation) | 2 | 1.10 | 2.0 | 10.3 |
| 10 | 10 | G12D | - | G12D (after operation) | 1.8 | 3.40 | 61.5 | 44.8 |
| 11 | 11 | G12V | - | wild (after operation) | 2 | 0.83 | 2.4 | 16.5 |
| 12 | 12 | wild | - | Q61R (after operation) | 2 | 34.40 | 19.7 | 85.2 |
| 13 | 13 | wild | - | wild (after operation) | 2 | 0.23 | 2.3 | 4.4 |
| 14 | 6 | wild | - | Q61H (after operation) | 2 | 40.40 | 1158.6 | 2.0 |
| 15 | 14 | wild | wild | G13D (after operation) | 2 | 1.80 | 9.6 | 45.1 |
| 16 | 9 | G12A | - | wild (after operation) | 3 | 0.60 | 1.5 | 3.4 |
| 17 | 15 | G12v | - | G12V (after operation) | 3 | 0.80 | 55.2 | 38.7 |
| 18 | 16 | wild | - | wild (after operation) | 2 | 1.60 | 22.3 | 169.8 |
| 19 | 17 | G12v | - | G12V (after operation) | 3.5 | 21.50 | 467.0 | 636.6 |
| 20 | 18 | wild | - | wild (before operation) | 4 | 2.20 | 2.2 | 2.0 |
| 21 | 19 | wild | - | wild (before operation) | 2 | 1.53 | 79.4 | 35.8 |
| 22 | 20 | G12v | - | G12V (after operation) | 4 | 22.40 | 433.7 | 8.1 |
| 23 | 21 | wild | - | wild (after operation) | 3 | 2.05 | n.d. | n.d. |

The coincident rate between KRAS gene mutation (codons 12 and 13) of a surgical specimen of a primary lesion and KRAS gene mutation in cfDNA was 81.8%. In addition, a correlation table of the KRAS gene mutation in the primary lesion and the KRAS gene mutation in cfDNA is shown in Table 6. In Table 6, "pDNA" means DNA which is extracted from a primary lesion. In the calculation of Cohen's kappa coefficient κ, the κ value became 0.58 when applying P (coincident rate) and Pe (coincident rate in a case where the results between two samples were accidentally coincident with each other) to κ(=(P-Pe)/(1-Pe).

**[Table 6]**

| cfDNA | pDNA | | Total |
|---|---|---|---|
| | Mutant | Wild type | |
| Mutant | 9 | 2 | 11 |
| Wild type | 2 | 9 | 11 |
| Total | 11 | 11 | 22 |

It should be noted that G13D (KRAS protein in which the 13-th glycine was replaced with aspartic acid) was detected from cfDNA of patients with ID number 8 and ID number 14 whose primary lesion and metastatic lesion were a wild type.
Furthermore, the tumor reduction effect was not observed even if cetuximab which is an EGFR inhibitor was administered to the patient with ID number 8. This result shows that the status of KRAS gene mutation of a tumor tissue does not necessarily become a predictive factor for therapeutic effect of an EGFR inhibitor. In contrast, the tumor of the patient with ID number 14 was reduced by greater than or equal to 20% from a CT scanning result. The tumor reduction rate (reduction effect) was calculated from the diameter of the tumor of a CT image. In a case where a plurality of tumor is dotted, the tumor reduction rate was calculated by adding the diameters of the tumors.

Consequently, KRAS gene mutation (G12A) was observed from circulating DNA similarly to the case of a primary lesion before surgery of resecting the primary lesion of the patient with ID number 9. However, G12A was not detected from circulating DNA after the surgery of resecting the primary lesion. Furthermore, the prognosis of this patient was good. Therefore, this suggests that it is possible to apply the detection of the KRAS gene mutation from cfDNA in serum or blood plasma to prognosis of a patient with cancer or to diagnosis of recurrence of cancer. In addition, this is also connected to early diagnosis by observing cfDNA in peripheral blood before the response of CEA or CA19-9 which is a cancer marker.

In the clinical analysis on patients with recurrent colorectal cancer in Example 1, mutated KRAS genes were observed also from serum which had been collected when the recurrence of cancer was diagnosed in all of the patients in whom the mutated KRAS genes were observed in a primary lesion. From this result, in diagnosis of the recurrence of cancer after resecting the primary lesion, it is possible to confirm the usefulness of identifying the mutated KRAS gene during circulation and confirm the existence of any recurrent tumor early (earlier than the existing biomarker such as CEA or CA 19-9 depending on patients) by checking the status of KRAS in blood of a subject who had received therapy with respect to the primary lesion, over time.

In addition, regarding the codon 61 of KRAS, samples in which the KRAS codons 12 and 13 were wild types in a primary tissue and serum were retrieved. As a result, Q61H was detected in the sample numbers 6 and 14, and Q61R was detected in the sample number 12. The sample numbers 6 and 14 correspond to an identical patient, and were changed to PD (progressiveness) after collecting the sample 14. Accordingly, it was found that the KRAS gene mutation of the codon 61 in serum also became a predictive factor for therapeutic effect of anti-EGFR antibody drug.

### INDUSTRIAL APPLICABILITY

The method for predicting sensitivity to an EGFR inhibitor according to the present invention uses a peripheral blood sample. For this reason, it is possible to predict sensitivity to an EGFR inhibitor in a less invasive manner and efficacy of the EGFR inhibitor with high accuracy without resection of a primary lesion or biopsy collection such as biopsy. From the viewpoint of low invasiveness or favorable accuracy, it is considered that the method for predicting sensitivity to an EGFR inhibitor according to the present invention is widely spread as an alternative method of fecal occult blood or the like in cancer screening.

While preferred embodiments of the invention have been described and shown above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A method for predicting sensitivity to an EGFR inhibitor, comprising:
(a) determining whether there is a KRAS gene-derived nucleic acid or a protein thereof in a blood sample which has been collected from a subject, and whether the KRAS gene-derived nucleic acid or the protein thereof in the blood sample is wild type or mutant; and
(b) determining that there is a high possibility that a tumor of the subject is sensitive to an EGFR inhibitor when a wild type KRAS gene-derived nucleic acid or a protein thereof is detected and no mutant KRAS gene-derived nucleic acid or a protein thereof is detected in the blood sample in the process (a), and determining that there is a high possibility that the tumor of the subject is not sensitive to the EGFR inhibitor in a case where a mutant KRAS gene-derived nucleic acid or the protein thereof is detected in the blood sample.

2. The method for predicting sensitivity to an EGFR inhibitor according to claim 1, wherein
even if a genotype of a KRAS gene-derived nucleic acid or a protein thereof which is detected from a tissue specimen or a cell specimen which has been collected from the tumor of the subject is different from a genotype of the KRAS gene-derived nucleic acid or the protein thereof which is detected from the blood sample, it is determined that there is a high possibility that the tumor of the subject is not sensitive to the EGFR inhibitor in a case where the mutant KRAS gene-derived nucleic acid or the protein thereof is detected in the blood sample which has been collected from the subject, in the process (b).

3. The method for predicting sensitivity to an EGFR inhibitor according to claim 1 or 2, wherein
the subject had received surgical resection treatment which had been performed on a tumor site in the past.

4. The method for predicting sensitivity to an EGFR inhibitor according to any one of claims 1 to 3, wherein
the subject had been administered with the EGFR inhibitor in the past.

5. The method for predicting sensitivity to an EGFR inhibitor according to claim 4, wherein
the subject had exhibited a drug tolerance to the EGFR inhibitor in the past.

6. The method for predicting sensitivity to an EGFR inhibitor according to claim 4 or 5, wherein
the blood sample is collected from a subject 60 days after administration of the EGFR inhibitor.

7. The method for predicting sensitivity to an EGFR inhibitor according to claim 1 or 2, wherein
the subject is a tumor patient who has received an antitumor therapy which is different from an EGFR inhibitor administration treatment after reception of the EGFR inhibitor administration treatment, and wherein
the blood sample is collected before the tumor patient receives the EGFR inhibitor administration treatment again.

8. The method for predicting sensitivity to an EGFR inhibitor according to claim 7, wherein
the antitumor therapy which is different from the EGFR inhibitor administration treatment is a therapy of administering a chemotherapeutic agent.

9. The method for predicting sensitivity to an EGFR inhibitor according to claim 8, wherein
the chemotherapeutic agent is one or more selected from the group consisting of fluorouracil, folinic acid, oxaliplatin, irinotecan, cytarabine, fludarabine, gemcitabine, hydroxyurea, methotrexate, bleomycin, chlorambucil, cisplatin, cyclophosphamide, doxorubicin, mitoxantrone, camptothecin, topotecan, teniposide, colcemid, colchicine, paclitaxel, vinblastine, vincristine, and tamoxifen.

10. The method for predicting sensitivity to an EGFR inhibitor according to claim 7, wherein
the antitumor therapy which is different from the EGFR inhibitor administration treatment is a radiation therapy.

11. The method for predicting sensitivity to an EGFR inhibitor according to claim 7, wherein
the antitumor therapy which is different from the EGFR inhibitor administration treatment is a therapy of administering a molecular target drug which is different kind from the EGFR inhibitor that has already been administered to the subject.

12. The method for predicting sensitivity to an EGFR inhibitor according to claim 11, wherein
the molecular target drug is one or more selected from the group consisting of cetuximab, panitumumab, bevacizumab, gefitinib, erlotinib, regorafenib, crizotinib, sunitinib, sorafenib, everolimus, trastuzumab, lapatinib, and rituximab.

13. The method for predicting sensitivity to an EGFR inhibitor according to claim 11 or 12, wherein
the antitumor therapy which is different from the EGFR inhibitor administration treatment is a combined therapy of the therapy of administering the molecular target drug and the therapy of administering the chemotherapeutic agent.

14. The method for predicting sensitivity to an EGFR inhibitor according to any one of claims 1 to 13, wherein
the tumor is a recurrent tumor.

15. The method for predicting sensitivity to an EGFR inhibitor according to any one of claims 1 to 13, wherein
the tumor is a metastatic lesion.

16. The method for predicting sensitivity to an EGFR inhibitor according to any one of claims 1 to 12, wherein
the tumor is a primary lesion.

17. The method for predicting sensitivity to an EGFR inhibitor according to any one of claims 1 to 16, wherein
the tumor is one or more selected from the group consisting of colorectal cancer, colon cancer, rectal cancer, lung cancer, liver cancer, breast cancer, ovarian cancer, prostate cancer, kidney cancer, esophageal cancer, head and neck cancer, uterine cancer, and cervical cancer.

18. The method for predicting sensitivity to an EGFR inhibitor according to any one of claims 1 to 18, wherein
the tumor exists in a plurality of sites in a body of the subject.

19. The method for predicting sensitivity to an EGFR inhibitor according to any one of claims 1 to 18, wherein
the mutant is one or more selected from the group consisting of G12A, G12C, G12D, G12R, G12S, G12V, G13D, G12S2, G13A, G13S, G13V, G13R, G13C, Q61H, Q61L, Q61R, A146T, andA146V of a KRAS protein.

20. The method for predicting sensitivity to an EGFR inhibitor according to any one of claims 1 to 19, wherein
the determination of the presence and absence of the KRAS gene-derived nucleic acid or the protein thereof in the blood sample and the determination whether the KRAS gene-derived nucleic acid or the protein thereof is wild type or mutant, are performed by checking whether the wild type KRAS gene-derived nucleic acid is detected or the mutant KRAS gene-derived nucleic acid is detected from a circulating DNA in the blood sample.

21. The method for predicting sensitivity to an EGFR inhibitor according to any one of claims 1 to 20, wherein
the blood sample is one of the group consisting of peripheral blood, serum, and blood plasma.

22. The method for predicting sensitivity to an EGFR inhibitor according to any one of claims 1 to 21, wherein
CEA in the blood sample is less than or equal to 5 ng/mL or the CA19-9 value in the blood sample is less than or equal to 37.0 U/mL.
